# EUROPEAN PATENT APPLICATION

(11) **EP 2 808 682 A1**
(43) Date of publication of application: **03.12.2014**
(21) Application number: 13169686.6
(22) Date of filing: 29.05.2013
(51) Int. Cl.: G01N 33/569, G01N 33/68, C12Q 1/37, G01N 33/573

(54) **Diagnosis and/or treatment of diseases, which are related to allergic reactions to Staphylococcus aureus**

(71) Applicant: Ernst-Moritz-Arndt-Universität Greifswald, 17487 Greifswald (DE)
(72) Inventor: Bröker, Barbara, 17489 Greifswald (DE); Engelmann, Susanne, 17489 Greifswald (DE); Hecker, Michael, 17509 Lubmin (DE); Kolata, Julia, 17489 Greifswald (DE); Steil, Leif, 17498 Dersekow-Hof (DE); Völker, Uwe, 17498 Neuenkirchen (DE); Link, Christopher, 23558 Lübeck (DE); Stentzel, Sebastian, 17491 Greifswald (DE); Bachert, Claus, 9830 Sint Martens-Latem (BE)
(74) Representative: Wablat Lange Karthaus

(57) **Abstract**

The invention suggests methods of diagnosis and therapy of diseases, which are related to allergic reactions to the *Staphylococcus aureus*and corresponding preparations. For diagnosis an interaction of at least one serine protease like protein of *Staphylococcus aureus*or a derived protein or peptide with a blood sample or a serum sample of a patient is analyzed. For the therapeutic aspect at least one serine protease like protein of *Staphylococcus aureus*or a derived protein or peptide is used as allergen in a systemic immune therapy approach.

SEQUENCE LISTING

<110>  Ernst-Moritz-Arndt Universitaet Greifswald

<120>  Diagnosis and/or treatment of diseases, which are related to
       allergic reactions to Staphylococcus aureus

<130>  PVA-25 588 EP

<160>  6

<170>  PatentIn version 3.5

<210>  1
<211>  235
<212>  PRT
<213>  Staphylococcus aureus

<400>  1

Met Asn Lys Asn Val Met Val Lys Gly Leu Thr Ala Leu Thr Ile Leu
1               5                   10                  15

Thr Ser Leu Gly Phe Ala Glu Asn Ile Ser Asn Gln Pro His Ser Ile
            20                  25                  30

Ala Lys Ala Glu Lys Asn Val Lys Glu Ile Thr Asp Ala Thr Lys Glu
        35                  40                  45

Pro Tyr Asn Ser Val Val Ala Phe Val Gly Gly Thr Gly Val Val Val
    50                  55                  60

Gly Lys Asn Thr Ile Val Thr Asn Lys His Ile Ala Lys Ser Asn Asp
65                  70                  75                  80

Ile Phe Lys Asn Arg Val Ser Ala His His Ser Ser Lys Gly Lys Gly
                85                  90                  95

Gly Gly Asn Tyr Asp Val Lys Asp Ile Val Glu Tyr Pro Gly Lys Glu
            100                 105                 110

Asp Leu Ala Ile Val His Val His Glu Thr Ser Thr Glu Gly Leu Asn
        115                 120                 125

Phe Asn Lys Asn Val Ser Tyr Thr Lys Phe Ala Asp Gly Ala Lys Val
    130                 135                 140

Lys Asp Arg Ile Ser Val Ile Gly Tyr Pro Lys Gly Ala Gln Thr Lys
145                 150                 155                 160

Tyr Lys Met Phe Glu Ser Thr Gly Thr Ile Asn His Ile Ser Gly Thr
                165                 170                 175

Phe Met Glu Phe Asp Ala Tyr Ala Gln Pro Gly Asn Ser Gly Ser Pro
        180                 185                 190

Val Leu Asn Ser Lys His Glu Leu Ile Gly Ile Leu Tyr Ala Gly Ser
    195                 200                 205

Gly Lys Asp Glu Ser Glu Lys Asn Phe Gly Val Tyr Phe Thr Pro Gln
    210                 215                 220

Leu Lys Glu Phe Ile Gln Asn Asn Ile Glu Lys
225                 230                 235

<210>  2
<211>  240
<212>  PRT
<213>  Staphylococcus aureus

<400>  2

Met Asn Lys Asn Val Val Ile Lys Ser Leu Ala Ala Leu Thr Ile Leu
1               5                   10                  15

Thr Ser Val Thr Gly Ile Gly Thr Thr Leu Val Glu Glu Val Gln Gln
            20                  25                  30

Thr Ala Lys Ala Glu Asn Asn Val Thr Lys Val Lys Asp Thr Asn Ile
        35                  40                  45

Phe Pro Tyr Thr Gly Val Val Ala Phe Lys Ser Ala Thr Gly Phe Val
    50                  55                  60

Val Gly Lys Asn Thr Ile Leu Thr Asn Lys His Val Ser Lys Asn Tyr
65                  70                  75                  80

Lys Val Gly Asp Arg Ile Thr Ala His Pro Asn Ser Asp Lys Gly Asn
                85                  90                  95

Gly Gly Ile Tyr Ser Ile Lys Lys Ile Ile Asn Tyr Pro Gly Lys Glu
            100                 105                 110

Asp Val Ser Val Ile Gln Val Glu Glu Arg Ala Ile Glu Arg Gly Pro
        115                 120                 125

Lys Gly Phe Asn Phe Asn Asp Asn Val Thr Pro Phe Lys Tyr Ala Ala
    130                 135                 140

Gly Ala Lys Ala Gly Glu Arg Ile Lys Val Ile Gly Tyr Pro His Pro
145                 150                 155                 160

Tyr Lys Asn Lys Tyr Val Leu Tyr Glu Ser Thr Gly Pro Val Met Ser
                165                 170                 175

Val Glu Gly Ser Ser Ile Val Tyr Ser Ala His Thr Glu Ser Gly Asn
            180                 185                 190

Ser Gly Ser Pro Val Leu Asn Ser Asn Asn Glu Leu Val Gly Ile His
        195                 200                 205

Phe Ala Ser Asp Val Lys Asn Asp Asp Asn Arg Asn Ala Tyr Gly Val
    210                 215                 220

Tyr Phe Thr Pro Glu Ile Lys Lys Phe Ile Ala Glu Asn Ile Asp Lys
225                 230                 235                 240

<210>  3
<211>  239
<212>  PRT
<213>  Staphylococcus aureus

<400>  3

Met Asn Lys Asn Ile Val Ile Lys Ser Met Ala Ala Leu Ala Ile Leu
1               5                   10                  15

Thr Ser Val Thr Gly Ile Asn Ala Ala Val Val Glu Glu Thr Gln Gln
            20                  25                  30

Ile Ala Asn Ala Glu Lys Asn Val Thr Gln Val Lys Asp Thr Asn Ile
        35                  40                  45

Phe Pro Tyr Asn Gly Val Val Ser Phe Lys Asp Ala Thr Gly Phe Val
    50                  55                  60

Ile Gly Lys Asn Thr Ile Ile Thr Asn Lys His Val Ser Lys Asp Tyr
65                  70                  75                  80

Lys Val Gly Asp Arg Ile Thr Ala His Pro Asn Gly Asp Lys Gly Asn
                85                  90                  95

Gly Gly Ile Tyr Lys Ile Lys Ser Ile Ser Asp Tyr Pro Gly Asp Glu
            100                 105                 110

Asp Ile Ser Val Met Asn Ile Glu Glu Gln Ala Val Glu Arg Gly Pro
        115                 120                 125

Lys Gly Phe Asn Phe Asn Glu Asn Val Gln Ala Phe Asn Phe Ala Lys
    130                 135                 140

Asp Ala Lys Val Asp Asp Lys Ile Lys Val Ile Gly Tyr Pro Leu Pro
145                 150                 155                 160

Ala Gln Asn Ser Phe Lys Gln Phe Glu Ser Thr Gly Thr Ile Lys Arg
                165                 170                 175

Ile Lys Asp Asn Ile Leu Asn Phe Asp Ala Tyr Ile Glu Pro Gly Asn
            180                 185                 190

Ser Gly Ser Pro Val Leu Asn Ser Asn Asn Glu Val Ile Gly Val Val
        195                 200                 205

Tyr Gly Gly Ile Gly Lys Ile Gly Ser Glu Tyr Asn Gly Ala Val Tyr
    210                 215                 220

Phe Thr Pro Gln Ile Lys Asp Phe Ile Gln Lys His Ile Glu Gln
225                 230                 235

<210>  4
<211>  239
<212>  PRT
<213>  Staphylococcus aureus

<400>  4

Met Asn Lys Asn Ile Ile Ile Lys Ser Ile Ala Ala Leu Thr Ile Leu
1               5                   10                  15

Thr Ser Ile Thr Gly Val Gly Thr Thr Val Val Asp Gly Ile Gln Gln
            20                  25                  30

Thr Ala Lys Ala Glu Asn Ser Val Lys Leu Ile Thr Asn Thr Asn Val
        35                  40                  45

Ala Pro Tyr Ser Gly Val Thr Trp Met Gly Ala Gly Thr Gly Phe Val
    50                  55                  60

Val Gly Asn His Thr Ile Ile Thr Asn Lys His Val Thr Tyr His Met
65                  70                  75                  80

Lys Val Gly Asp Glu Ile Lys Ala His Pro Asn Gly Phe Tyr Asn Asn
                85                  90                  95

Gly Gly Gly Leu Tyr Lys Val Thr Lys Ile Val Asp Tyr Pro Gly Lys
            100                 105                 110

Glu Asp Ile Ala Val Val Gln Val Glu Glu Lys Ser Thr Gln Pro Lys
        115                 120                 125

Gly Arg Lys Phe Lys Asp Phe Thr Ser Lys Phe Asn Ile Ala Ser Glu
    130                 135                 140

Ala Lys Glu Asn Glu Pro Ile Ser Val Ile Gly Tyr Pro Asn Pro Asn
145                 150                 155                 160

Gly Asn Lys Leu Gln Met Tyr Glu Ser Thr Gly Lys Val Leu Ser Val
                165                 170                 175

Asn Gly Asn Ile Val Thr Ser Asp Ala Val Val Gln Pro Gly Ser Ser
            180                 185                 190

Gly Ser Pro Ile Leu Asn Ser Lys Arg Glu Ala Ile Gly Val Met Tyr
        195                 200                 205

Ala Ser Asp Lys Pro Thr Gly Glu Ser Thr Arg Ser Phe Ala Val Tyr
    210                 215                 220

Phe Ser Pro Glu Ile Lys Lys Phe Ile Ala Asp Asn Leu Asp Lys
225                 230                 235

<210>  5
<211>  238
<212>  PRT
<213>  Staphylococcus aureus

<400>  5

Met Asn Lys Asn Ile Ile Ile Lys Ser Ile Ala Ala Leu Thr Ile Leu
1               5                   10                  15

Thr Ser Val Thr Gly Val Gly Thr Thr Val Val Glu Gly Ile Gln Gln
            20                  25                  30

Thr Ala Lys Ala Glu His Asn Val Lys Leu Ile Lys Asn Thr Asn Val
        35                  40                  45

Ala Pro Tyr Asn Gly Val Val Ser Ile Gly Ser Gly Thr Gly Phe Ile
    50                  55                  60

Val Gly Lys Asn Thr Ile Val Thr Asn Lys His Val Val Ala Gly Met
65                  70                  75                  80

Glu Ile Gly Ala His Ile Ile Ala His Pro Asn Gly Glu Tyr Asn Asn
                85                  90                  95

Gly Gly Phe Tyr Lys Val Lys Lys Ile Val Arg Tyr Ser Gly Gln Glu
            100                 105                 110

Asp Ile Ala Ile Leu His Val Glu Asp Lys Ala Val His Pro Lys Asn
        115                 120                 125

Arg Asn Phe Lys Asp Tyr Thr Gly Ile Leu Lys Ile Ala Ser Glu Ala
    130                 135                 140

Lys Glu Asn Glu Arg Ile Ser Ile Val Gly Tyr Pro Glu Pro Tyr Ile
145                 150                 155                 160

Asn Lys Phe Gln Met Tyr Glu Ser Thr Gly Lys Val Leu Ser Val Lys
                165                 170                 175

Gly Asn Met Ile Ile Thr Asp Ala Phe Val Glu Pro Gly Asn Ser Gly
            180                 185                 190

Ser Ala Val Phe Asn Ser Lys Tyr Glu Val Val Gly Val His Phe Gly
        195                 200                 205

Gly Asn Gly Pro Gly Asn Lys Ser Thr Lys Gly Tyr Gly Val Tyr Phe
    210                 215                 220

Ser Pro Glu Ile Lys Lys Phe Ile Ala Asp Asn Thr Asp Lys
225                 230                 235

<210>  6
<211>  239
<212>  PRT
<213>  Staphylococcus aureus

<400>  6

Met Asn Lys Asn Ile Ile Ile Lys Ser Ile Gly Ala Leu Thr Ile Leu
1               5                   10                  15

Thr Ser Ile Thr Gly Val Gly Thr Thr Met Val Glu Gly Ile Gln Gln
            20                  25                  30

Thr Ala Lys Ala Glu Asn Thr Val Lys Gln Ile Thr Asn Thr Asn Val
        35                  40                  45

Ala Pro Tyr Ser Gly Val Thr Trp Met Gly Ala Gly Thr Gly Phe Val
    50                  55                  60

Val Gly Asn His Thr Ile Ile Thr Asn Lys His Val Thr Tyr His Met
65                  70                  75                  80

Lys Val Gly Asp Glu Ile Lys Ala His Pro Asn Gly Phe Tyr Asn Asn
                85                  90                  95

Gly Gly Gly Leu Tyr Lys Val Thr Lys Ile Val Asp Tyr Pro Gly Lys
            100                 105                 110

Glu Asp Ile Ala Val Val Gln Val Glu Glu Lys Ser Thr Gln Pro Lys
        115                 120                 125

Gly Arg Lys Phe Lys Asp Phe Thr Ser Lys Phe Asn Ile Ala Ser Glu
    130                 135                 140

Ala Lys Glu Asn Glu Pro Ile Ser Val Ile Gly Tyr Pro Asn Pro Asn
145                 150                 155                 160

Gly Asn Lys Leu Gln Met Tyr Glu Ser Thr Gly Lys Val Leu Ser Val
                165                 170                 175

Asn Gly Asn Ile Val Ser Ser Asp Ala Ile Ile Gln Pro Gly Ser Ser
            180                 185                 190

Gly Ser Pro Ile Leu Asn Ser Lys His Glu Ala Ile Gly Val Ile Tyr
        195                 200                 205

Ala Gly Asn Lys Pro Ser Gly Glu Ser Thr Arg Gly Phe Ala Val Tyr
    210                 215                 220

Phe Ser Pro Glu Ile Lys Lys Phe Ile Ala Asp Asn Leu Asp Lys
225                 230                 235

## Description

The present invention relates to a method for diagnosis of diseases, which are related to allergic reactions to *Staphylococcus aureus,* as well as diagnostic preparations and therapeutic applications.

Asthma is one of the most common chronic diseases throughout the world. About 300 million people currently suffer from Asthma. Two forms of asthma can be distinguished: The allergic (exogenous) asthma and the so-called non-allergic (endogenous) asthma, which is also known as idiopathic or intrinsic asthma. Both forms are diseases of the respiratory system. Allergic asthma is mediated by IgE. It leads to the release of mast cell products which results in bronchial constriction and impaired respiration. Allergic asthma is triggered and maintained by typical inhalation allergies such as pollens, animal and herbal dusts, animal proteins and chemicals. On the contrary, non-allergic asthma is not caused by known allergens. Infections of the respiratory system, intolerance to drugs, and exposure to poisons are discussed as causes for this asthma form. Nevertheless, in at least 10 % of the endogenous, non-allergic asthma cases the causative agent is still unknown. These are referred to as cases of idiopathic or intrinsic asthma. However, an immune reaction to *Staphylococcus aureus*(*S. aureus*) could possibly explain a subgroup of intrinsic asthma, as IgE antibodies *against S.aureus* enterotoxins have recently been described in a group of severe asthmatics (Bachert, C., et al., Specific IgE against Staphylococcus aureusenterotoxins: an independent risk factor for asthma. J Allergy ClinImmunol, 2012. 130(2): p. 376 - 81 e. 8).

Chronic rhinosinusitis (CRS) is a heterogeneous group of inflammatory diseases of the nasal cavities. There are two forms of CRS: First CRS with nasal polyposis (CRSwNP) and second CRS without polyps (CRSsNP). A clinical study conducted in 23 centers throughout Europe showed that about 11 % of the European population suffers from CRS. This study also revealed that CRS was strongly associated with asthma, especially in patients with CRSwNP (Bachert, C. and N. Zhang, Chronic rhinosinusitis and asthma: novel understanding of the role of IgE'above atopy'. J Intern Med, 2012. 272(2): p. 133 - 43). Factors within CRSwNP tissue which predict asthma comorbidity have been identified as IL5, SE-IgE, total IgE and ECP (Bachert, C., et al., see above).

*S. aureus* is a micro-organism colonizing about 20 % of mankind persistently as a commensal. The preferred niches are the interior naresand the pharynx. Besides this, *S.aureus* causes a broad range of infections like pneumonia, osteomyelitis, skin and soft tissue infections and sepsis.

There is a set of 21 identified superantigens (SAgs) in *S. aureus.* Genetic analysis of *S. aureus* clinical isolates, including whole genome sequencing, has shown that around 80 % of all *S. aureus* clinical isolates harbor SAg genes, on average five to six (Holtfreter, S., et al., egc-Encoded superantigens from Staphylococcus aureusare neutralized by human sera much less efficiently than are classical staphylococcal enterotoxins or toxic shock syndrome toxin. Infect Immun, 2004.72(7): p. 4061-71;Jarraud, S., et. al., egc, ahighly prevalentoperon of enterotoxin gene, forms a putative nursery of superantigens in Staphylococcus aureus. J Immunol, 2001.166(1): p. 669 - 77). Now SAgs are being discussed to modulate the immune response in chronic rhinosinusitis and thus cause this disease as well as asthma (Bachert, C. and N. Zhang, see above).

Clinical evidence points to an important role of allergic reactions to *S. aureus* (IgE-mediated hypersensitivities of type 1) in chronic severe airway inflammation. Especially patients with chronic rhinosinusitis accompanied by nasal polyposis are more frequently colonized by *S. aureus* than subjects without polyps (Gevaert, P., et al., Organization of secondary lymphoid tissue and local IgE formation to Staphylococcus aureusenterotoxins in nasal polyp tissue. Allergy, 2005.60(1): p. 71-9). Bachert et al. (see above) have shown that nasal polyposis together with colonization of the polyps by *S.aureusis* strongly associated with endogenous idiopathic asthma. IgE-antibodies with specificity for *S. aureus* enterotoxins (SAEs), namely the staphylococcal enterotoxin A (SEA), the staphylococcal enterotoxin B (SEB), the staphylococcal enterotoxin C (SEC), and the toxic shock syndrome toxin 1 (TSST1) indicate an allergic reaction to *S. aureus* in some patient groups. Patients with IgE-antibodies specific for certain superantigens, namely SAEs, had a higher risk to develop idiopathic asthma than patients without such antibodies (see for example Gevaert, P., et al., see above).

Besides the endogenous asthma, there is also an allergic tendency in other diseases associated with *S. aureus* colonization and/or infection, for example, in atopic dermatitis (AD) and allergic rhinitis. 90 % of AD patients are colonized by *S. aureus* (Reginald, K., et al., Immunoglobulin E antibody reactivity to bacterial antigens in atopic dermatitis patients. ClinExp Allergy, 2011.41(3): p. 357 - 69).

Another group of S. *aureus* proteins is the so-called serine protease-like proteins (Spl). This is a group of six proteins (SplA-F) encoded on the *spl*operon (Reed, S. B., et. al., Molecular characterization of a novel Staphylococcus aureusserine protease operon. Infect Immun, 2001. 69(3): p. 1521 - 7). The Spls are not well characterized and their substrates are not known. For SpIB and SpIC casein has been suggested as substrate (Dubin, G., et al., Enzymaticactivity of the Staphylococcus aureusSplBserine protease is induced by substrates containing the sequence Trp-Glu-Leu-Gln. J MolBiol, 2008. 379(2): p. 343-56).

Non-allergic or idiopathic asthma, chronic rhinosinusitis and nasal polyps as well as aspirin hypersensitivity are common chronic inflammatory conditions of the respiratory system. They tend to take a severe disease course. The situation is similar in the case of AD, an allergic disease affecting the skin. Nevertheless, the causes of these conditions are not known. The diseases are notoriously difficult to treat because they do not respond well to standard allergy treatments. Moreover, cases that are treatment refractory are common. There is no causative treatment of these diseases. Thus, the object of the present invention is to provide an effective approach to treat diseases, which are related to allergic reactions to *S. aureus,* especially for a treatment of non-allergic or idiopathic asthma, aspirin hypersensitivity, chronic rhinosinusitis and nasal polyps as well as AD.

Additionally, there is no sensitive and specific tool to diagnose allergic reactions to *S. aureus.* Currently allergic reactions to *S. aureus* are diagnosed by measuring IgE binding to merely four selected *S. aureus* SAEs: SEA, SEB, SEC und TSST1. However, only one out of three clinical *S. aureus* strains are endowed with these SAEs, and 20 % of *S. aureus* clinical isolates from the nose of patients do not own any SEA gene at all, whereby not all cases of endogenous idiopathic asthma, nasal polyposis or AD can be explained and whereby it is unlikely that the above-mentioned SEAs are the triggers of hypersensitivity type I reactions, i.e. allergies to *S. aureus* (Holtfreter, S., et al., Clonal distribution of superantigengenes in clinical Staphylococcus aureusisolates. J ClinMicrobiol, 2007. 45(8): p. 2669 - 80). Thus a further object of the present invention is to provide a sensitive and specific approach to diagnose allergic reactions to *S. aureus.*

These objects are solved by methods for diagnosis and treatment of diseases, which are related to allergic reactions to *S.aureus,* diagnostic and pharmaceuticalpreparations and a method to prepare a medicament for treatment of such diseasesas it is set forth in the independent claims. Preferred embodiments of the methods and preparations are described in the dependent claims.

The claimed subject matters are based on the findings of the inventors that *S.aureus* serine proteases, especially SplA (SEQ ID NO. 1), SplB (SEQ ID NO. 2), SplC (SEQ ID NO. 3), SplD (SEQ ID NO. 4), SplE (SEQ ID NO. 5), and SplF (SEQ ID NO. 6) act as allergens in humans resulting in diverse allergic diseases, especially non-allergic or idiopathic asthma, chronic rhinosinusitis, nasal polyps, aspirin hypersensitivity and AD. There is strong evidence that these serine proteases act as triggers and pace-makers of such allergic diseases in susceptible individuals. The claimed diagnosis and treatment is based on the well-supported assumption that the serine proteases SplA-F are major allergens of *S. aureus.* In susceptible individuals exposure to these proteases triggers allergic reactions and maintains them. These allergic inflammatory reactions are the cause of frequent chronic diseases, which often take a severe course and are often refractory to symptomatic treatment strategies: Non-allergic or idiopathic asthma, aspirin hypersensitivity, rhinosinusitis with or without nasal polyps, and AD. Up until now, the pathogenesis of these diseases is largely unknown. The present invention enables for the first time a sensitive and specific diagnosis and a causal therapy.

As a first aspect the present invention claims a method for diagnosis of diseases, which are related to allergic reactions to *S.aureus.* The inventive method is characterized by an analysis or monitoring of an interaction of at least one serine protease like protein of *S.aureus* or a derived protein or peptide with the patient's body, especially with the immune system of the patient, by analyzing a blood sample or a serum sample of the patient.

Preferably serum antibody binding to the serine protease like proteins or derived proteins orpeptides is measured in the course of the diagnostic method. It is especially preferred to analyze IgE-and/or IgG4-antibodies of the patient. Advantageously, the inventive method is based on an assay for the determination of IgE- and/or IgG4-antibodies with specificity for one or several *S. aureus* serine proteases or serine protease like proteins. IgE molecules are causative agents of allergies of the type I, also known as atopic allergies. Generally, they occur when the organism has developed an allergy to a certain antigen, called allergen, and trigger characteristic allergic immune reactions, e.g. mast cell degranulation (this type of allergy is TH2 driven in human response). The more allergen-specific IgE is present, the stronger the allergic reactions are. Consequently, the measurement of IgE-antibodies with specificity for *S. aureus* serine protease like proteins is particularly suitable for diagnosis of allergic diseases related to *S. aureus* according to the inventive method. IgG4-antibodies are so-called neutralizing antibodies. They are expressed after long-term exposure to allergens and counteract the IgE-driven inflammation. Their neutralizing capacity is due to binding to the specific allergen and to their inability to activate pro-inflammatory effector functions in the immune system.IgE- and IgG4-production both depend on IL4, a cytokine elaborated by TH2 cells. IgG4-producingB cells may switch to IgEproduction in response to repeated allergen contact (Aalberse,R. C. and T.A. Platts-Mills, How do weavoid developing allergy: modifications of the TH2 response from a B-cell perspective. J Allergy ClinImmunol, 2004. 113(5): p. 983 - 6). Therefore, also the analysis of IgG4-antibodies with specificity for one or several S. aureus serine protease like proteins is particularly suitable for diagnosis of allergic diseases according to the inventive method.

The serine protease like protein which is the basis of the inventive approach, is at least one protein selected from the group consisting of SplA(SEQ ID NO. 1), SplB (SEQ ID NO. 2), SplC (SEQ ID NO. 3), SplD (SEQ ID NO. 4), SplE (SEQ ID NO. 5), and SplF (SEQ ID NO. 6). By the term serine protease like protein these serine proteases are comprised. Moreover, derived proteins or peptides, which are essentially based on the amino acid sequences of these proteases are also comprised. For example, the derived proteins or peptides may comprise only a part of the amino acid sequence of the original protein and/or some of the amino acids may be exchanged by similar amino acids. The resulting amino acid sequences of the derived proteins or peptides lacking enzymatic activity may preserve at least 90 % of the original protein sequences. With respect to the derived proteins orpeptides it may be especially advantageous if the derived protein or peptide preserves some or most or all of the allergenic epitopes of the original protein, especially in view of diagnostic applications, and/or if the derived protein or peptide lacks enzymatic activity, especially in view of therapeutic applications as described below.

In a preferred embodiment of the claimed diagnostic and therapeutic methods the serine protease like proteins or the derived proteins or peptides are recombinant proteins or peptides. Advantageously the proteins or peptides may be produced by known genetic engineering methods. By genetic engineering the proteins or peptides may be produced in large scale if necessary. Moreover, the proteins or peptides may be adapted and engineered according to the requirements of the inventive method or application. For example, it may be advantageous to alter or to delete the enzymatic activity of the recombinant protein or peptide and/or to alter or to weaken or to strengthen the allergenic properties of the recombinant protein or peptide.

Before use of the proteins or peptides for the inventive diagnostic or therapeutic applications it may be advantageous to purify the proteins or peptides in order to enhance the specificity of the method.

The inventive diagnostic method may be performed as ELISA-procedure (enzyme-linked immune-sorbent assay). The at least one serine protease like protein of *S. aureus* or the derived protein or peptide may be adsorbed to a solid phase and exposed to the patient's serum. Then antibody binding can be measured with standard methods. Alternatively, the allergens, namely the serine protease like proteins or derived proteins or peptides, may be coupled to fluorescent particles, which enable multiple measurements in a single serum sample. This approach may be performed in a multiplex assay, using, for example, the known Luminex®system. Moreover, the inventive diagnostic test could also be developed in the form of a protein array. Numerous different *S. aureus* serine protease like proteins or derived proteins or peptides may be coupled to a solid phase in an array format. Upon incubation with the patient's serum, the antibody binding to all these proteins can be determined simultaneously in a multiplex test. Another possibility is to first immobilize the antibodies, especially IgE-antibodies, from the patient's serum on a solid phase using, for example, affinity material for IgE, for example the known materialIgE-AviQuant® of the company PLS-Design. Then the *S. aureus* serine protease like proteins or derived proteins or peptide, respectively allergens, which are labeled by genetic engineering e.g. with a streptavidin tag, are added. The binding of the labeled allergens may be visualized by using streptavidin for detection. Further assays for the measurement of serum antibody binding to serine protease like proteins of *S. aureus* or derived proteins or peptides are possible.

Further the invention comprises the use of at least one serine protease like protein of *S. aureus* or a derived protein orpeptide for the manufacture of a diagnostic preparation for diagnosis of diseases, which are related to allergic reactions to *S. aureus.* The diagnostic preparation may be designed for ELISA-like procedures or other assays for the measurement of serum antibody binding as mentioned above, for example including labeling of the serine protease like proteins or derived proteins or peptides. With respect to further features of the claimed use it is referred to the description above.

Moreover, the diagnostic preparation may be designed to be intra-dermally introduced by pricking the skin. Minute amounts of the proteins or peptides are introduced intra-dermally in the course of the inventive diagnostic method. Then the local skin reaction is monitored. Especially a swelling at the pricksite appearing after 30 to 90 minutes will be diagnostic for a positive reaction, especially a *S. aureus* allergy. Especially in view of a skin prick application it may be advantageous to apply genetically modified proteins without enzymatic activity. There may be concerns that the enzymatic activity of the serine proteases might have adverse effects upon intradermal application. By the use of derived proteins or peptides without enzymatic activity or with altered enzymatic activity, which can easily be derived by recombinant gene technology, this problem may be solved.

For the skin prick approach it is especially preferred to use recombinant proteins or peptides derived from *S. aureus* serine proteins SplA-F, because by using genetic engineering there are no problems in producing large scale amounts of the proteins or peptides. Moreover, there are no problems with inhomogeneity of the proteins or peptides. Preferably, the recombinant *S. aureus* serine proteases or derived proteins or peptides are generated and purified using GMP protocols (good manufacturing practice).

The inventive diagnostic method is much more sensitive and more specific in the diagnosis of diseases which are related to allergic reactions to *S. aureus* than already known methods. Especially the diagnosis of asthma, especially non-allergic or idiopathic asthma, and/or chronic rhinosinusitis, and/or nasal polyps, and/or aspirin hypersensitivity, and/or AD is strongly improved by the inventive diagnostic method.

Moreover, the invention comprises a diagnostic preparation for diagnosis of diseases, which are related to allergic reactions to *S. aureus,* wherein the diagnostic preparation comprises at least one serine protease like protein of *S. aureus* or a derived protein or peptide, as described above. Preferably, the diagnostic preparation also comprises at least one pharmaceutically acceptable vehicle.

The further main aspect of the present invention is a therapeutic approach on the basis of the findings of the inventors. Thus, the invention comprises the use of at least one serine protease like protein of *S. aureus* or a derived protein or peptide for the manufacture of a medicament for treatment of diseases, which are related to allergic reactions to *S. aureus,* by a systemic immune therapeutic approach. It is known in the medical field that the systemic immune therapy is merely the only causal intervention strategy to terminate allergic reactions. In this therapeutic approach the allergens, which trigger the disease symptoms, are regularly applied to the patient. In the beginning minute amounts of the allergens are thus applied. Gradually the applied amounts are increased. Frequently this is a long-term therapy lasting several years before resolution or significant amelioration of a symptom. Generally there are two methods for systemic immune therapy. Firstly, the substances are applied intra-dermallyand/or subcutaneously and/or intra-muscularly. Secondly, the substances are administered sublingually. Both methods have been shown to be highly effective in different forms of allergy. Up to now there was no systemic immune therapy approach for *S. aureus* allergies. As long as superantigens are considered to be the main molecular triggers of *S. aureus* allergies, a systemic immune therapy was not possible because of the known toxic effects of the superantigens. In contrast the inventive approach uses at least one serine protease like protein of *S. aureus* or derived protein or peptide as allergen to be applied in the course of the systemic immune therapy. There are no known specific toxic effects of these proteins or peptides. Moreover, the enzymatic activity of the serine protease like protein of *S. aureus* or derived protein or peptide to be used for therapy can be eliminated by genetic engineering. Consequently, a systemic immune therapy can be done by use of these proteins or peptides based on the findings of the inventors, that these proteins or peptides are major allergens of *S. aureus.* With respect to further details of this aspect of the invention it is also referred to the above description.

The medicament for the systemic immune therapy comprises besides the serine protease like protein or a derived protein or peptide preferably at least one pharmaceutically acceptable vehicle. Preferably, the medicament is prepared to be applied intra-dermally and/or sublingually to the patient. The systemic immune therapy may be designed to apply the serine protease like protein or derived proteins or peptides a number of times beginning with a minute amount gradually increasing. Generally the therapy is designed to be applied for a longer term, preferably at least several months up to years.

It is especially preferred to employ the *S. aureus* allergens, namely the serine protease like proteins or derived proteins or peptides as recombinant proteins. The recombinant proteins may be generated by known genetic engineering methods and expressed in harmless bacteria or by other known methods and purified for example from bacterial culture supernatants by known biochemical methods. It is preferred to adhere to GMP standards for the production of the recombinant serine protease like protein or derived protein or peptide. The recombinant production of the protein or peptide is especially appropriate, because *S. aureus* releases a number of potent toxins that could otherwise contaminate the protein preparations and have adverse effects. In addition, such contaminating *S. aureus* toxins could induce an inflammatory reaction, which would counteract the therapeutic effects of the systemic immune therapy.

It may be preferred to genetically modify the serine protease like proteins or derived proteins or peptides, for example to eliminate their protease activity while preserving most allergenic epitopes. This can be achieved by known recombinant gene technology.

Generally, the causal therapy of allergy by systemic immune therapy encompasses two components: Firstly, the allergens, which are the triggers and pace-makers of the allergic symptoms, and secondly the particular preparation and/or application method which minimize inflammation and thereby facilitate the development of antigen-specific immune tolerance, which extinguishes the allergic inflammation in response to the bacteria. Therefore, it is preferred to avoid pro-inflammatory adjuvants to the therapeutic preparation in order to minimize inflammation during the course of the inventive systemic immune therapy. This is a fundamental difference to classical immunization schemes aiming a protection against infection wherein pro-inflammatory adjuvants are frequently added to the immunizing antigen.

Finally, the invention comprises a pharmaceutical preparation for treatment of diseases, which are related to allergic reactions to *S. aureus,* by a systemic immune therapy, wherein the preparation comprises at least one serine protease like protein of *S. aureus* or a derived protein or peptide as described above. By the pharmaceutical preparation respectively medicament it is possible for the first time to treat the causes of non-allergic or idiopathic asthma, aspirin hypersensitivity, chronic rhinosinusitis, nasal polyps, and AD especially in those cases that are caused or exacerbated by an allergic reaction against *S. aureus.*

Further features and advantages of the invention read from the following description of the experimental part in conjunction with the figures, wherein each of the features may be realized individually or in combination with each other.

In the figures it is shown:
- Fig. 1: graphical analysis of *S. aureus* specific IgG4 in sera of healthy carriers and non-carriers of *S. aureus;*
- Fig. 2: graphical analysis of enzyme-linked immuno-sorbent assays (ELISA) for IgE and IgG4 binding to SplC, SplD and TSST-1 in sera from carriers and non-carriers of *S. aureus;*
- Fig. 3: graphical analysis of PBMC (peripheral blood mononuclear cells) stimulated with heat-inactivated recombinant antigens SplC, SplD and Hla (α-Toxin);
- Fig. 4: graphical analysis of frequencies of SplC- and SplD-specific T cells in carriers and non-carriers of *S. aureus;*
- Fig. 5: graphical analysis of release of cytokines of PBMC stimulated with heat-inactivated Hla or SplD; and
- Fig. 6: graphical analysis of cytokines released by SplD-specific T cell clones.

### Experimental part

### a. Most adults have IgG4 in their serum that binds to proteins released by S. aureus.

The humoral immune response of five healthy *S. aureus* carriers to extracellular *S. aureus* proteins was examined by two-dimensional (2D) immune proteomics.

For antigen preparation, bacteria were inoculated in tryptic soy broth (TSB) to an optical density at 540 nm of 0.05 and cultivated in 100 ml cultures of TSB at 37 °C and 180 rpm. 3.5 h after the bacterial culture had entered the stationary phase, cultures were harvested, the extra-cellular proteins were extracted and protein concentration was determined as previously described (Holtfreter, S., et al., Human immune proteome in experimental colonization with Staphylococcus aureus. Clin Vaccine Immunol, 2009. 16(11): p. 1607-14).Extra-cellular proteins were stored in aliquots at -80°C. Serum and plasma samples were obtained from five *S. aureus* carriers; they were stored in aliquots at -80°C.

Two-dimensional polyacrylamide gel electrophoresis (2-DE) with mini 2-DE gels (2D gels) and 2-DE immunoblots (2D-IBs) were performed as described (Holtfreter, S., et al., see above).In short, isoelectric focusing was performed with 7 cm Immobiline Dry Strips (GE Healthcare, Munich, Germany). The pH range of 6-11 was chosen for analysis because most extra-cellular proteins resolved in this range, while protein A was excluded so that unspecific antibody binding, which obscured a large part of the blot at a pH range of 4-7, was avoided. The separated staphylococcal proteins were blotted onto a PVDF membrane (Immobilon-P, Millipore, Billerica, USA) with 1.33 mA/cm² for 2 h (graphite blotter MilliBlot; Millipore, Billerica, USA) and incubated with the corresponding human sera at 1:10,000 dilution. Binding of IgG4 was detected by horseradish peroxidase (HRP)-conjugated goat anti-human IgG4 (Invitrogen, Darmstadt, Germany) and visualized with an ECL substrate (SuperSignal West Femto Maximum Sensitivity Substrate, Pierce, Rockford, USA). Three independent experiments were performed for each carrier.

Whereas all five *S. aureus* carriers showed strong binding of total serum IgG to the bacterial antigens, IgG4 binding to the same bacterial antigens was highly variable.

These findings were corroborated by examining the binding of serum antibodies (total IgG and IgG4) in a larger cohort of healthy adults, 16 *S. aureus* carriers and 16 non-carriers. For this a partially automated one-dimensional (1D) immunoblot-based assay system was employed (Simon^{™}, ProteinSimple, Santa Clara, CA, USA). Briefly, acrylamide gel matrix, dilutions of bacterial antigen (1 µg/µl), plasma (1:50), secondary goat-anti-human IgG4-HRP antibody (1:100; Invitrogen) and luminol substrate (ProteinSimple, Santa Clara, CA, USA) were prepared and loaded into SIMON. According to the predefined experimental conditions, SIMON performed 1D-immunoblots in capillaries. Raw data weresubsequently analyzed with Compass software (ProteinSimple, Santa Clara, CA, USA) and GraphPad Prism 5 (GraphPad, La Jolla, CA, USA).

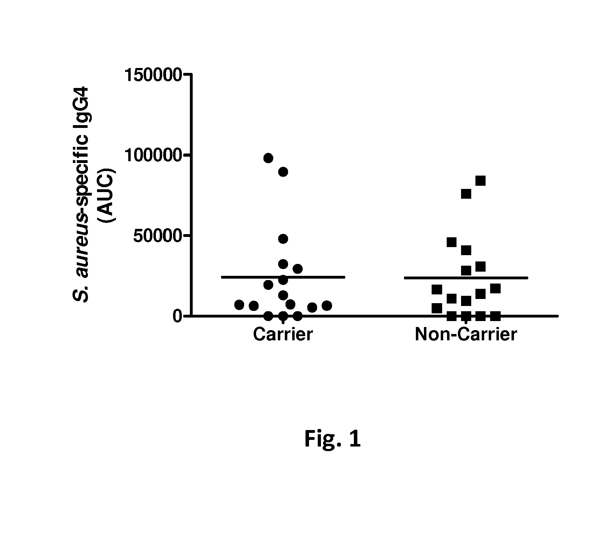

Approximately 80% of healthy adults exhibit measurable serum antibodies of the IgG4 subclass binding to *S. aureus* antigens, 20% did not. The binding intensity varied strongly between the individuals as shown in **Fig. 1****.** This figure illustrates the results of an automated 1-dimensional immunoblots (SIMON) with extracellular *S*. *aureus* proteins. The immunoblots were used to determine *S*. *aureus*-specific IgG4 in sera of healthy adultS. aureuscarriers and non-carriers. The proteins were separated by their molecular weight, incubated with the corresponding serum. IgG4 binding was detected via an anti-human-IgG4-HRP antibody preparation and a luminol substrate. Besides a great variance in binding intensity, nearly 80 % of all adults showed *S*. *aureus*-specific serum IgG4.

### b. The major IgG4-binding proteins were identified as the S. aureus serine protease-like proteins SpIC and SpID.

Bacterial proteins bound by serum antibodies were identified by overlaying the 2D immunoblot pictures with the corresponding 2D-PAGE. For protein identification 100 µg of extra-cellular proteins were loaded onto 11 cm Immobiline Dry Strips (GE Healthcare, Munich, Germany) with the pH range 6-11. After second dimension protein separation gels were stained with Flamingo^{®} Fluorescent Gel Stain (BioRad, Munich, Germany) according to the manufacturer's instructions, except for fixation, which was performed twice for 1 h. Gels were scanned using a Typhoon 9400 scanner (GE Healthcare, Munich, Germany) in the fluorescence acquisition mode (532 nm) at a resolution of 100 µm. Subsequently, peptides were prepared for mass spectrometry by MALDI-MS by trypsin digestion as previously described (Holtfreter, S., et al., see above). The MALDI-TOF measurement of spotted peptide solutions was carried out on a Proteome-Analyzer 4700/4800 (Applied Biosystems, Foster City, CA, USA) as reported. Database searches were performed using the GPS explorer software version 3.6 (build 3329) with an organism-specific database. The combined MS and MS/MS peak lists were searched against a *S*. *aureus*8325 protein database obtained from the ENTREZ genome database site (ftp://ftp.ncbi.nih.gov/genomes/Bacteria/) and a database containing protein sequences derived from the genome sequences of all completely sequenced *S*. *aureus* strains and, moreover, all additional protein sequences of *S*. *aureus* (continuously updated from www.uniprot.org) using the Mascot search engine version 2.104 (Matrix Science, London, UK). The following search criteria were applied: Carbamidomethylation (C) and oxidation (M) were set as variable modifications; a peptide mass tolerance of ± 50 ppm and a fragment mass tolerance of ± 0.55 Da were used; the peptide charge state of +1 was accepted for the precursor peptides; the maximum number of missed cleavages was set to 1. The Mowse score for a significant identification of a protein spot had to exceed a value of 50, which corresponds to a *P*-value of 0.05 (Kolata, J., et al., Distinctive patterns in the human antibody response to Staphylococcus aureus bacteremia in carriers and non-carriers. Proteomics, 2011. 11(19): p. 3914-27).

Whereas total serum IgG binding on 2-dimensional immunoblots was observed to a variety of bacterial proteins, *S*. *aureus* specific IgG4 binding was strongest at a molecular mass of 32 kDa and 34 kDa. In some sera IgG4 binding was exclusively observed in this region. The corresponding *S*. *aureus* proteins were identified by mass spectrometry to be SpIC and SpID. Hence SpIC and SpID were identified to be the dominant IgG4-binding proteins of *S*. *aureus.*

This was corroborated by the 1-dimensional immunoblot analysis of serum IgG4 binding to S. *aureus* proteins in 16 *S. aureus* carriers and 16 non-carriers. If IgG4 binding was taking place at all, it was always highly abundant in a region corresponding to a molecular mass of 32 kDa and 34 kDa, which is the respective size of the Spls. IgG4 binding to bacterial proteins was monitored in all sera and binding to bacterial proteins with a molecular mass of 32 to 34 kDa was observed in 13 of 16 carriers and 12 of 16 non-carriers. This supports the results of the analysis of the 2-dimensional immunoblots, namely that Spl proteins are the dominant IgG4-binding proteins of S. *aureus.*

### c. The species S. aureus harbors six serine protease like genes, spIA-F.

Up to six *S*. *aureus*serine protease genes are encoded on the *spl*-operon (Reed, S.B., et al., Molecular characterization of a novel Staphylococcus aureus serine protease operon. Infect Immun, 2001. 69(3): p. 1521-7; Dubin, G., et al., Enzymatic activity of the Staphylococcus aureus SpIB serine protease is induced by substrates containing the sequence Trp-Glu-Leu-Gln. J Mol Biol, 2008. 379(2): p. 343-56).The gene loci encoding each of the *S*. *aureus* serine protease like proteins, namely SpIA (SEQ ID NO. 1), SpIB (SEQ ID NO. 2), SpIC (SEQ ID NO. 3), SpID (SEQ ID NO. 4), SpIE (SEQ ID NO. 5), and SpIF (SEQ ID NO. 6), are highly conserved between the sequenced S. *aureus* strains (at least 95 % homology) (Zdzalik, M., et al., Prevalence of genes encoding extracellular proteases in Staphylococcus aureus - important targets triggering immune response in vivo. FEMS Immunol Med Microbiol, 2012. 66(2): p. 220-9).

However, the sequences of Spls A-F, which are encoded by different gene loci, are rather variable (Table 1).

**Tab. 1: Comparison of the serine protease amino acid sequences(Reed, S.B., et al., Molecular characterization of a novel Staphylococcus aureus serine protease operon. Infect Immun, 2001. 69(3): p. 1521-7).**

| | SPlA | SplB | SplC | SplD | SplE | SplF | V8 |
|---|---|---|---|---|---|---|---|
| SplA | 100 | 47.7 | 50.0 | 43.9 | 43.9 | 44.8 | 33.3 |
| SplB | | 100 | 62.9 | 54.4 | 56.2 | 53.9 | 30.4 |
| SplC | | | 100 | 49.8 | 50.4 | 51.0 | 30.6 |
| SplD | | | | 100 | 67.4 | 94.6 | 32.7 |
| SplE | | | | | 100 | 67.8 | 36.4 |
| SplF | | | | | | 100 | 32.5 |
| V8 | | | | | | | 100 |

### d. The S. aureus proteins SpIC and SplD were produced as recombinant proteins and purified.

For the investigation of the interplay between S. aureusserine protease like proteins (Spls) and the immune system, the SpIC and SplD were produced in recombinant form in E. coli. They were expressed with a C-terminal Strep-tag, added by genetic engineering methods, for purification by affinity chromatography on columns coated with streptactin. To avoid nonspecific immune cell activation by E. coli-LPS, the resulting protein preparations were subsequently depleted of lipopolysaccharide (LPS) using EndoTrap^{®} red columns (Hyglos, Heidelberg, Germany) according to the manufacturer's instructions. The column material binds with high affinity to LPS and the protein of interest can be eluted while LPS remains bound in the column. Two rounds of purification yielded in final LPS concentrations below 10 ng LPS per mg protein.

The amino acid sequences of recombinant SpIC and SpIC without signal peptide are as follows, wherein a methionine was added to the N-terminus (bold) and the Strep-tag sequence (SAWSHPQFEK) was attached to the C-terminus (bold):

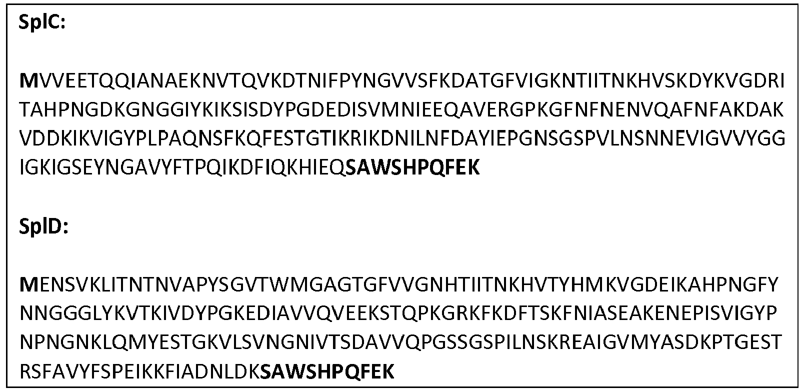

The sequences were taken from the genome of the fully sequenced *S*. *aureus* strain USA300. The signal peptides - as predicted by UniProt (http://www.uniprot.org/) - were removed.

### e. The subclass composition of serum antibodies that bind to recombinant S. aureusSpIC and SpID is shifted towards IgG4 in around 50% of healthy adults.

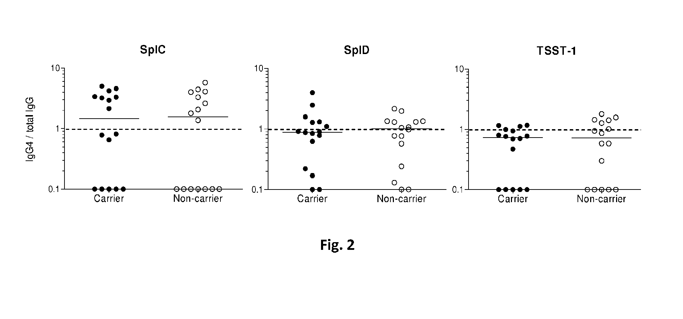

Serum antibody binding to recombinant SpIC and SpID was investigated by an enzyme linked immuno-sorbent assay (ELISA). Recombinant TSST-1 served as control. Sera of 32 healthy blood donors, 16 S. aureus carriers and 16 non-carriers, were analyzed for total serum IgG binding to SpIC, SpID, and TSST-1. Moreover, the binding of serum antibodies of the subclass IgG4 was also measured by ELISA. The IgG4/IgG ratio for binding was shifted towards IgG4 in half of the tested sera for SpIC (Fig. 2). On the other hand, there were also individuals who did not elaborate serum IgG4 with specificity for the tested bacterial antigens (**Fig. 2**). **Fig. 2** shows the results of an enzyme-linked immune sorbent assays (ELISA) for IgG and IgG4 binding to SpIC, SpID and TSST-1. The assays were performed with sera from 16 carriers and 16 non-carriers. Serumwas diluted in blocking buffer (PBS/Tween, 2% milk powder) (serial dilutions 1:5; 1:50 to 1:31250) and tested in duplicate. Total IgG binding was detected with goat anti-human IgGantibodies(Jackson ImmunoResearc) conjugated with HRP, diluted in blocking buffer to a concentration of 10 ng/ml. Binding of IgG4 was detected using goat anti-human IgG4 antibodies conjugated with HRP (Invitrogen) diluted in blocking buffer to a concentration of 1 ng/ml. ELISA plates (Maxisorp, Thermo Scientific) were coated with 2 µg/ml µl antigen over night at 4 °C. After washing three times with PBS/Tween20 (0,05%) each well was blocked with 100 µl blocking buffer for one hour. 50 µl/well of serum dilutions were incubated for 1 hour at 100 rpm and the ELISA plates were washed three times as previously described. 50 µl of the diluted secondary antibody were added to each well and incubated for 1 hour. After three rounds of washing, 50 µl/well substrate solution wasadded for 10 min and the chromogenic reaction was stopped with 20 µl/well 2 N H₂SO₄. The signal was measured at 450 nm.

Experiments were performed in duplicates; mean values are depicted. Area under the curve was calculated for each serum and individual ratios of IgG4/IgG-binding are depicted. Carrier sera are represented by open circles; non-carrier sera by filled circles.

### f. Serum IgE with specificity for S. aureusSpIC and SpIDis found in patients with cystic fibrosis, atopic dermatitis, asthma, and chronic rhinosinusitis with nasal polyps.

IgE binding to SpIC and SpID was studied in sera of patients with cystic fibrosis (CF), atopic dermatitis (AD) and asthma using an ELISA assay as described above with the following modifications: Sera were diluted 1:100 in blocking buffer (PBS/10% fetal calf serum). IgE binding was measured by incubation with mouse anti-human IgE antibodies at a concentration of 10 µg/ml followed by incubation with rabbit anti-mouse IgG antibodies coupled with HRPat 3 µg/ml (secondary and tertiary antibody preparations were from Invitrogen). All five patients mounted an IgE response to SpIDand four out of five mounted an IgE response to SpIC (Table 2).

**Tab. 2: Serine protease specific IgE in patient sera.**

| **Patient** | **Anti-SpIC-IgE** | **Anti-SpID-IgE** |
|---|---|---|
| 1 (CF) | yes | yes |
| 2 (CF) | yes | yes |
| 3 (AD) | yes | yes |
| 4 (Asthma) | yes | yes |
| 5 (Asthma) | no | yes |

### g. Human adults harbor S. aureusSpIC- and SpID-responsive human T cells in the blood.

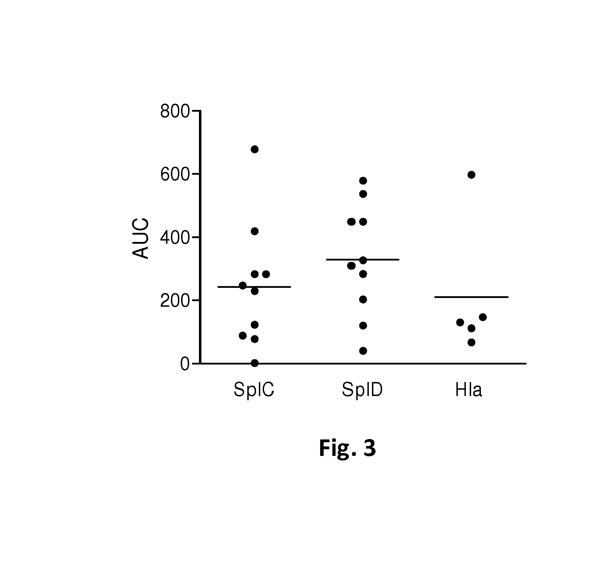

Peripheral blood mononuclear cells (PBMC) from ten adult human volunteers were incubated with recombinant SpIC and SpID at increasing concentrations. The cells from most volunteers responded with a robust, concentration-dependent proliferation as measured by the incorporation of ³H-methyl-thymidine using known methods. This indicates that SpIC-and SpID-reactive T cells are present in the blood of most human adults as shown in **Fig. 3**.SpIC and SpID induced a robust proliferative response in PBMC. For the assay PBMC from ten healthy blood donors were isolated and stimulated with heat inactivated (65°C, 45 min) recombinant antigens SpIC, SpID and α-toxin (Hla), which served as control. Cells were incubated with serially diluted antigens (10 - 0,08µg/ml) for 7 d. Proliferation was determined by ³H-methyl-thymidin incorporation. Area under the curve (AUC) was calculated for each donor.

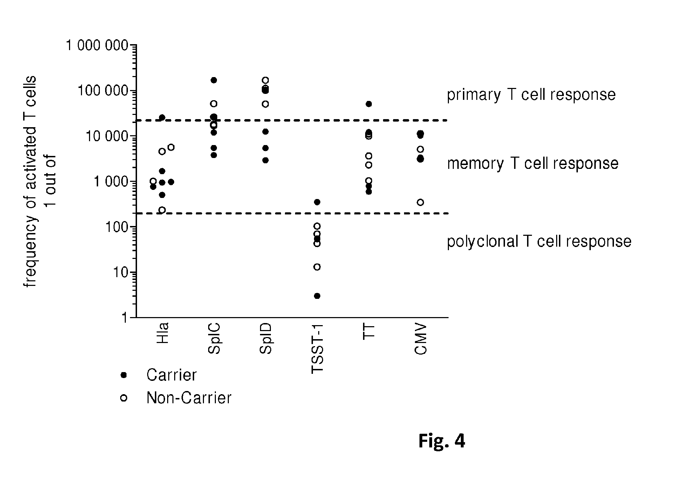

The frequency of SpIC- and SpID-specific T cells in the peripheral blood was assessed in nine healthy adult volunteers by limiting dilution assays. Irradiated autologous antigen-presenting cells were pulsed with 10 µg/ml antigen (SpIC or SpID) and incubated (37°C, 5 % CO₂) with serially diluted T cell suspensions resulting in an input of between 10,000 to 78 T cells per culture. Supplementation with low doses of IL-2 (10 IU/ml) enhanced the proliferation of antigen-specific T cell clones. Proliferation was quantified by ³H-methyl-thymidine incorporation after 10 days. The fraction of non-proliferating cultures was determined for each T cell number of input, and subsequently, the frequency of antigen-specific T cells was calculated from a regression curve. Frequencies of SpIC- and SpID-specific T cells ranged from 1 out of 3.000 to 1 out of 170.000 T cells as shown in **Fig. 4**.SpIC- and SpID-specific T cells are present atfrequencies typical for memory T cells. This can be inferred from the observation that T cells responsive to the typical recall antigens tetanus toxoid (TT) and antigens from cytomegaly virus (CMV) are of similar frequency. In contrast, the superantigen TSST-1 activated a much larger fraction of T cells such as it is expected of a superantigen **(****Fig. 4**).For the limiting dilution assays (LDA) autologous feeder cells were stimulated with 10 µg/ml of heat-inactivated antigen and serially diluted T cells (10⁴-78 T cells per culture) were added. Cells were incubated for 10 d in total, and supplemented with IL-2 at d 5 and d 10 (10 IU/ml and 50 IU/ml). Proliferation was determined by ³H-methyl-thymidine incorporation. For calculation of T cell frequencies, the number of non-proliferative cultures per T cell concentration was determined. According to the Poisson distribution, a fractionof 0,368 of non-proliferative cultures is equal to the limiting dilution. Thus, regression equations were solved with f(x)=0,368. Frequencies of antigen-specific T cells from carriers are depicted with open circles; cells from non-carriers with filled circles.

### h. A subset of SpIC- and SpID-specific human T cells generates a cytokine profile typical of an allergic reaction.

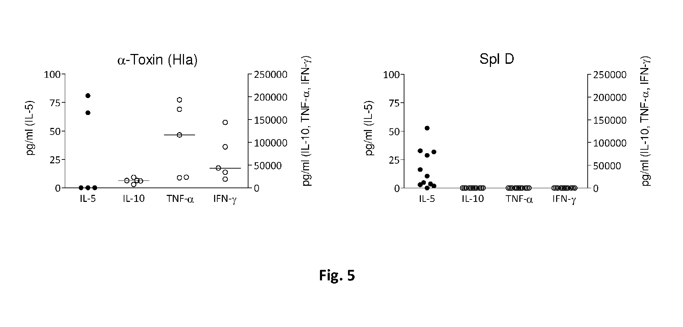

Peripheral blood mononuclear cells (PBMC) from five to ten adult human volunteers were incubated with recombinant SpIC, SpID and α-toxin at 5 µg/ml. After 7 d the cytokines released by the activated cells were analysed by flow cytometry. The investigated cytokine panel covered the main cytokines which characterize TH1, TH2, TH9, TH17, TH22 and Tregs. Analysis of the cytokine response of these SpIC-and SpID- stimulated T cells revealed secretion of the TH2 cytokine IL-5 (shown for SpIDin **Fig. 5**). There was almost no IL-10, TNF-α nor IFN-γ in these supernatants. The blood cell reaction to α-toxin was very different in that IL-10, TNF-α and IFN-γ were secreted at high concentrations (**Fig**. **5**). This shows that SpIC-and SpID-trigger a specific response in T cells. Such responses are typical of allergies of type 1. For the assay PBMC from five to ten healthy blood donors were stimulated with heat-inactivated (65°C, 45 min) α-toxin (right panel) or SpID (left panel) at concentrations of 5 µg/ml for 7 d. Cell culture supernatants were taken and analysed by flow cytometry for release of cytokines with flexible cytokine bead array (Becton Dickinson) according to the instruction manual. In contrast to the pro-inflammatory cytokines induced by α-toxin (e.g. IFN-γ), SpID-stimulated PBMC responded selectively with TH2 cytokines like IL-5.Every circlerepresents the concentration of a cytokine secreted in cell culture by the peripheral blood cells from one individual. The left Y-axis relates to the measured concentrations of IL5 (full circles),the right Y-axis to the measured concentrations of IL-10, TNF-α and IFN-γ (open circles).

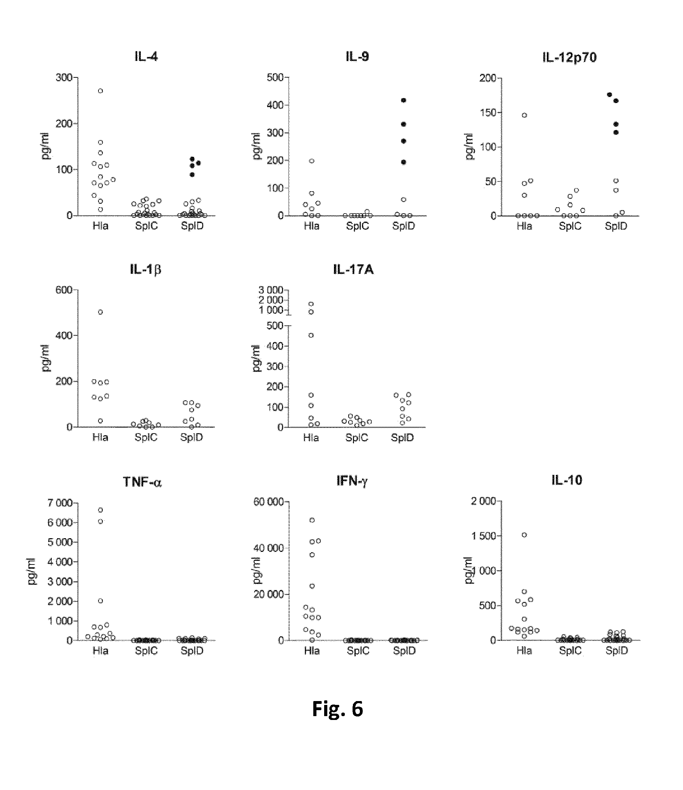

SpIC- and SpID-specific T cell clones were activated with their respective antigens for 72 h. T cell clones responsive to the staphylococcal α-toxin (Hla) served as controls. The released cytokines in the supernatants were analyzed by a flow cytometric bead assay. The investigated cytokine panel covered the main cytokines which characterize TH1, TH2, TH9, TH17, TH22 and Tregs. It was observed that half of the SpID-specific T cell clones produced the cytokines IL-4, IL-9 and IL-12p70. These are characteristic of TH2 and TH9 cells. Small amounts of IL-1β and IL-17 were noticed as well, but TNF-α and IFN-γ concentrations were below the detection limits. This is remarkable, because other well-known *S*. *aureus* virulence factors, like α-toxin (Hla), induced the specific T cell clones to release the pro-inflammatory cytokines TNF-α and IFN-γ in very large amounts (**Fig. 6**).Secretion of cytokines typically for regulatory T cells (Tregs), e.g. IL-10, were not observed in the Spl-specific T cell clones. Hence, a survey of *S*. *aureus*-reactive T cell clones revealed that a subset of T cell clones with specificity for SpIC and SpID is unique in that these T cells selectively secrete cytokines corresponding to a Th2/Th9 profile, namely IL-4, IL-5 and IL-9 (shown in black circles in Fig. 6). This supports the assumption that SpIC and SpID stimulation favors an allergic immune response profile even in healthy adults.

To obtain antigen-specific T cell clones, limiting dilution assays (LDA) were performed. Therefore, autologous feeder cells were stimulated with 10 µg/ml of heat-inactivated antigen, SpIC, SpID, or Hla, and co-incubated with serially diluted T cells (10⁴-78 T cells per culture). Cells were incubated for 10 d in total, and supplemented with IL-2 at d 5 and d 10 (10 IU/ml and 50 IU/ml). Growing T cell clones were stimulated with their respective antigen again and 72 h later, supernatants were obtained for analysis of cytokines. Cytokines were determined by flow cytometry with the flexible cytokine bead array (Becton Dickinson) according to the instruction manual. Every circle represents the cytokine concentration elaborated by an individual T cell clone specific for the respective antigen as shown. Filled circles represent four independent T cell clones, specific for SpID, which showed the most pronounced pro-allergic cytokine profiles.

### i. Conclusions

The results make a strong case for the concept that *S*. *aureus* serine protease like proteinsact as allergens in humans. The concept is based on the following findings:
- Many known allergens are proteases.
- Some individuals develop a strong IgG4-antibody response against *S*. *aureus* proteins. This is not directed against all *S*. *aureus* products with the same intensity, but IgG4 binding is strongest to SpIC and SpID. It isassumedthat adults with a strong IgG4 antibody binding to Spl proteins have an atopic predisposition.
- Healthy adults harbor T lymphocytes in their blood that specifically respond to SpIC or SpID. A significant subset of these T cells produces cytokines upon stimulation which are typical of allergic reactions of type 1: IL-4, IL-5 und IL-9.
- On the other hand it is remarkable that these Spl-specific T cells - in salient contrast to T cells with specificity for other *S*. *aureus* proteins - do not release cytokines that would counteract an allergic reaction: TNF-α, IFN-γ, IL-10. This is very different, for example, in α-toxin-reactive T cells.
- From the previous points it follows that the serine protease like proteinsof *S. aureus* favor the development of an immune response with a Th2 profile. A Th2-dominated immune response carries a high risk of development of allergic symptoms. Hence it is expected that the serine protease like proteinsSpIA-F act as triggers and pace-makers of allergies in susceptible individuals.
- In fact, allergic individuals have in their serum IgE-antibodies with specificity for SpIC and SpID.

From the above findings it is concluded that the serine protease like proteinsSpIA-F are major allergens of *S*. *aureus.* Exposure to these proteases triggers allergic reactions and maintains them in susceptible individuals and these allergic inflammatory reactions are the cause of frequent chronic diseases like non-allergic or idiopathic asthma, aspirin hypersensitivity,rhinosinusitis with or without nasal polyps, and AD. The findings lead to the concept of the invention to take serine protease like proteins of *S*. *aureus* or derived proteins or peptides as targets for diagnosis and treatment of diseases, which are related to allergic reactions to *S*. *aureus.* The first main aspect of the invention is the diagnosis of such diseases by analyzing an interaction of *S. aureus* serine protease like proteins or derived proteins or peptides with the patient's body, especially with serum antibodies of the patient. The second main aspect of the invention is a therapeutic application based on systemic immune therapy, wherein small amounts of *S. aureus* serine protease like proteins or derived proteins or peptidesare administered to the patient, especially by intra-dermal and/or sublingual administration.

### Protein sequence information

Theprotein sequences are one allelic form each of the serine protease like proteins A-F as they are present in the genome of *S. aureus* strain USA300 according to the database UniProt (http://www.uniprot.org/).
NO 1:*Staphylococcus aureus* serine protease like protein A (SpIA); Database accession no: Q2FFS9 
MNKNVMVKGLTALTILTSLGFAENISNQPHSIAKAEKNVKEITDATKEPYNSVVAFVGGT
GVVVGKNTIVTNKHIAKSNDIFKNRVSAHHSSKGKGGGNYDVKDIVEYPGKEDLAIVHVH
ETSTEGLNFNKNVSYTKFADGAKVKDRISVIGYPKGAQTKYKMFESTGTINHISGTFMEFDAYAQPGNSGS
PVLNSKHELIGILYAGSGKDESEKNFGVYFTPQLKEFIQNNIEK
 NO 2:*Staphylococcus aureus* serine protease like protein B (SpIB); Database accession no:Q2FFT0 
MNKNVVIKSLAALTILTSVTGIGTTLVEEVQQTAKAENNVTKVKDTNIFPYTGVVAFKSA
TGFVVGKNTILTNKHVSKNYKVGDRITAHPNSDKGNGGIYSIKKIINYPGKEDVSVIQVE
ERAIERGPKGFNFDNVTPFKYAAGAKAGERIKVIGYPHPYKNKYVLYESTGPVMSVEGS
SIVYSAHTESGNSGSPVLNSNNELVGIHFASDVKNDDNRNAYGVYFTPEIKKFIAENIDK
 NO 3: *Staphylococcus aureus* serine protease like protein C (SpIC); Database accession no: Q2FFT1 
MNKNIVIKSMAALAILTSVTGINAAVVEETQQIANAEKNVTQVKDTNIFPYNGVVSFKDATGFVIGKNTIIT
NKHVSKDYKVGDRITAHPNGDKGNGGIYKIKSISDYPGDEDISVMNIEEQAVERGPKGFNFNENVQAFNF
AKDAKVDDKIKVIGYPLPAQNSFKQFESTGTIKRIKDNILNFDAYIEPGNSGSPVLNSNNEVIGVVYGGIGKI
GSEYNGAVYFTPQIKDFIQKHIEQ
 NO 4: *Staphylococcus aureus* serine protease like protein D (SpID); Database accession no: Q2FFT2 
MNKNIIIKSIAALTILTSITGVGTTVVDGIQQTAKAENSVKLITNTNVAPYSGVTWMGAGTGFVVGNHTIIT
NKHVTYHMKVGDEIKAHPNGFYNNGGGLYKVTKIVDYPGKEDIAVVQVEEKSTQPKGRKFKDFTSKFNIA
SEAKENEPISVIGYPNPNGNKLQMYESTGKVLSVNGNIVTSDAVVQPGSSGSPILNSKREAIGVMYASDKP
TGESTRSFAVYFSPEIKKFIADNLDK
 NO 5: *Staphylococcus aureus* serine protease like protein E (SpIE); Database accession no: Q2 F FT3 
MNKNIIIKSIAALTILTSVTGVGTTVVEGIQQTAKAEHNVKLIKNTNVAPYNGVVSIGSGTGFIVGKNTIVTN
KHVVAGMEIGAHIIAHPNGEYNNGGFYKVKKIVRYSGQEDIAILHVEDKAVHPKNRNFKDYTGILKIASEA
KENERISIVGYPEPYINKFQMYESTGKVLSVKGNMIITDAFVEPGNSGSAVFNSKYEVVGVHFGGNGPGN
KSTKGYGVYFSPEIKKFIADNTDK
 NO 6: *Staphylococcus aureus* serine protease like protein F (SpIF); Database accession no: Q2FFT4

MNKNIIIKSIGALTILTSITGVGTTMVEGIQQTAKAENTVKQITNTNVAPYSGVTWMGAGTGFVVGNHTII
TNKHVTYHMKVGDEIKAHPNGFYNNGGGLYKVTKIVDYPGKEDIAVVQVEEKSTQPKGRKFKDFTSKFNI
ASEAKENEPISVIGYPNPNGNKLQMYESTGKVLSVNGNIVSSDAIIQPGSSGSPILNSKHEAIGVIYAGNKPS
GESTRGFAVYFSPEIKKFIADNLDK

## Claims

1. Method for diagnosis of diseases, which are related to allergic reactions to *Staphylococcus aureus,* **characterized in that** an interaction of at least one serine protease like protein of *Staphylococcus aureus* or a derived protein or peptide with a blood sample or a serum sample of a patient is analyzed.

2. Method according to claim 1, **characterized in that** the diseases are asthma, especially non-allergic or idiopathic asthma, and/or chronic rhinosinusitiswith or without nasal polyps and/or aspirin hypersensitivity, and/or atopic dermatitis.

3. Method according to claim 1 or 2, **characterized in that** serum antibody binding to the serine protease like proteins or derived proteins or peptides is measured as interaction.

4. Method according to claim 3, **characterized in that** the serum antibodies are IgE- and/or IgG4-antibodies.

5. Method according to any one of the preceding claims, **characterized in that** the at least one serine protease like protein is at least one selected from the group consisting of SpIA (SEQ ID NO. 1), SpIB (SEQ ID NO. 2), SpIC (SEQ ID NO. 3), SpID (SEQ ID NO. 4), SpIE (SEQ ID NO. 5) and SpIF (SEQ ID NO. 6).

6. Method according to any one of the preceding claims, **characterized in that** the at least one serine protease like protein or derived protein or peptide is a recombinant protein or peptide.

7. Method according to any one of the preceding claims, **characterized in that** the at least one serine protease like protein or derived protein or peptide is genetically modified, especially modified to preserve or alter some or most or all allergenic epitopes and/or to lack enzymatic activity.

8. Method according to any one of the preceding claims, **characterized in that** the at least one serine protease like proteinor derived protein or peptide is purified.

9. Use of at least one serine protease like protein of *Staphylococcus aureusor* a derived protein or peptide for the manufacture of a diagnostic preparation for diagnosis of diseases, which are related to allergic reactions to *Staphylococcus aureus.*

10. Use according to claim 9, further **characterized by** at least one feature of at least one of claims 2 to 8.

11. Use according to claim 9 or claim 10, **characterized in that** the at least one serine protease like protein or derived protein or peptide is to be introduced intra-dermally and the local reaction of the patient's body is monitored, especially by using the skin prick method.

12. Diagnostic preparation for diagnosis of diseases, which are related to allergic reactions to *Staphylococcus aureus,* **characterized in that** the preparation comprises at least one serine protease like protein of *Staphylococcus aureus* or a derived protein or peptide.

13. Diagnostic preparation of claim 12, further **characterized by** at least one feature of at least one of claims 2 to 8.

14. Diagnostic preparation according to claim 12 or claim 13, **characterized in that** the preparation further comprises at least one pharmaceutically acceptable vehicle.

15. Diagnostic preparation according to any one of claims 12 to 14, **characterized in that** the at least one serine protease like protein or derived protein or peptide is to be introduced intra-dermally and the local reaction of the patient's body is monitored, especially by using the skin prick method.

16. Use of at least one serine protease like protein of *Staphylococcus aureus* or a derived protein or peptide for the manufacture of a medicament for treatment of diseases, which are related to allergic reactions to *Staphylococcus aureus,* by a systemic immune therapy.

17. Use according to claim 16, further **characterized by** at least one feature of at least one of claims 2, 5, 6, 7 and 8.

18. Use according to claim 16 or claim 17, **characterized in that** the medicament further comprises at least one pharmaceutically acceptable vehicle.

19. Use according to any one of claims 16 to 18, **characterized in that** the medicament is prepared to be applied intra-dermally and/or subcutaneously and/or intra-muscularly and/or sublingually to the patient.

20. Use according to any one of claims 16 to 19, **characterized in that** the at least one serine protease like protein of *Staphylococcus aureus* or derived protein or peptide is prepared to be applied a number of times beginning with a minute amount gradually increasing.

21. Use according to any one of claims 16 to 20, **characterized in that** the systemic immune therapy minimizes inflammation.

22. Pharmaceutical preparation for treatment of diseases, which are related to allergic reactions to *Staphylococcus aureus,* by a systemic immune therapy, **characterized in that** the preparation comprises at least one serine protease like protein of *Staphylococcus aureus* or a derived protein or peptide.

23. Pharmaceutical preparation of claim 22, further **characterized by** at least one feature of at least one of claims 17 to 21.
